# EUROPÄISCHE PATENTSCHRIFT

(11) **EP 1 127 552 B1**
(45) Veröffentlichungstag und Bekanntmachung des Hinweises auf die Patenterteilung: **14.06.2006**
(21) Anmeldenummer: 00128679.8
(22) Anmeldetag: 29.12.2000
(51) Int. Cl.: A61B 18/14

(54) **Ablationskatheter zur Erzeugung linearer Läsionen in Herzmuskelgewebe**
Ablation catheter for producing linear lesions in the heart muscle
Cathéter pour créer une lésion linéaire dans le muscle cardiaque

(30) Priorität: 25.02.2000 DE 10008918
(43) Veröffentlichungstag der Anmeldung: 29.08.2001
(73) Patentinhaber: BIOTRONIK GmbH & Co. KG, 12359 Berlin (DE)
(72) Erfinder: Töllner, Thomas, 12047 Berlin (DE); Maxfield, Michelle, 10967 Berlin (DE); Richter, Jan Helge, 12355 Berlin (DE)
(74) Vertreter: Lindner-Vogt, Karin L.

(56) Entgegenhaltungen:
- WO-A-96/00039
- US-A- 5 398 683
- US-A- 5 637 090
- US-A- 5 685 878
- US-A- 5 730 127

## Beschreibung

Die Erfindung betrifft einen Ablationskatheter zur Erzeugung linearer Läsionen in Herzmuskelgewebe mit einem langgestreckten Katheterkörper, der ein proximales und ein distales Ende aufweist, und mit einer linearen, im wesentlichen zylindrischen Ablationselektrode am distalen Ende des Katheterkörpers.

Zum Hintergrund der Erfindung ist festzuhalten, daß die Katheterablation eine vermehrt eingesetzte Therapie zur Behandlung bestimmter Arrhythmien ist. Dabei wird an einer bestimmten Stelle im Herzmuskelgewebe mit Hilfe der Ablationselektrode des Katheters eine Läsion - also eine Art Gewebeabtragung oder -vernarbung - erzeugt, um dort die für die Arrhythmien verantwortliche, fehlerhafte elektrische Reizleitung zu unterbrechen. Bei der Behandlung atrialen Flatterns oder Flimmerns werden insbesondere lineare Läsionen erzeugt.

Die Energieeinbringung in das Herzmuskelgewebe über die Ablationselektrode erfolgt dabei entweder mit Hochspannungs-Gleichstrom oder - was in jüngerer Zeit aufgrund der Probleme mit Gleichstrom bevorzugt wird - mit elektromagnetischer Strahlung im Radiofrequenzbereich. Grundsätzlich ist es im übrigen bei einer Ablationstherapie wichtig, die Ablationselektrode präzise innerhalb des Herzens zu plazieren.

Herkömmliche Ablationskatheter weisen nun in einer ersten Grundvariante unipolare, punktuell wirksame Ablationselektroden auf, so daß diese zwischen einzelnen Ablationsschritten mehrfach versetzt werden müssen, falls lineare, also langgestreckte Läsionen erzeugt werden sollen. Dieser Vorgang ist zeitaufwendig und wegen der Ortsunsicherheit der Lage der Ablationselektrode innerhalb des Herzens verhältnismäßig unpräzise. Zur Verbesserung in dieser Hinsicht sind als zweite Variante bereits Ablationskatheter bekannt, die multipolare, punktuell wirksame Elektroden aufweisen, deren einzelne Pole von einem speziell dafür geeigneten Ablations-Steuergerät sequentiell anzusteuern sind.

Aus der US-A-5,676,693 ist ein Ablationskatheter zur Erzeugung linearer Läsionen bekannt, der mittels einer aus dem Katheter austretenden, leitfähigen Flüssigkeit und mehrerer darinliegender Elektroden eine längliche, quasi flüssige Elektrode erzeugt.

Aus der US-A-5,800,428 ist ein Ablationskatheter zur Erzeugung linearer Läsionen bekannt, der eine dünne, drahtartige, langgestreckte Ablationselektrode am distalen Ende aufweist. In einer speziellen Ausführungsform sind am distalen Ende mehrere miteinander kombinierte Drähte als Ablationselektroden vorgesehen, die zwei- oder dreidimensional aufgespreizt sind. Die Elektrodendrähte können dabei elektrisch voneinander isoliert sein.

Schließlich ist aus der US-A-5,676,662 ein Ablationskatheter zur Erzeugung linearer Läsionen bekannt, bei dem die Ablationselektrode aus einem spiralförmig auf Abstand gewickelten, teilweise in den Katheterkörper eingebetteten Leiter besteht. Dieser ist auch auf der Außenseite von einer Isolationsschicht abgedeckt, die entlang eines längsparallel verlaufenden Streifens ausgespart ist, um die Außenseiten der spiralförmig gewickelten Leiter freizulegen. Diese bilden in diesem Bereich die ablativ wirksame, lineare Zone der Elektrode.

Bei diesem Ablationskatheter ist es vorgesehen, einen Teil der Wicklungen der Ablationselektrode getrennt ansteuerbar zu machen, so daß die abgetrennten Leiterstücke als Abtastelektrode zur Erfassung elektrokardiologischer Signale vor oder nach dem eigentlichen Ablationsvorgang - zum sogenannten "mapping" - geeignet ist. Durch diese Konfiguration können verschiedene Abschnitte des spiralförmigen Elektrodenabschnittes verschiedene Abschnitte des Herzmuskelgewebes ohne Verschiebung der Spiralelektrode erfassen oder abtasten.

Ferner offenbart US-A-5,398,683 einen Ablationskatheter gemäß dem Oberbegriff des Anspruchs 1.

Den bekannten Ablationselektroden ist gemeinsam, daß die genaue Positionierbarkeit aufgrund der begrenzten oder überhaupt nicht gegebenen Abtastmöglichkeiten elektrokardiologischer Signale zu wünschen übrig läßt.

Davon ausgehend liegt der Erfindung die Aufgabe zugrunde, einen Ablationskatheter der gattungsgemäßen Art so weiterzubilden, daß die Ablationselektrode eine verbesserte Positionierbarkeit aufweist.

Diese Aufgabe wird durch die Merkmale des kennzeichnenden Teils des Anspruchs 1 gelöst.

Aufgrund dieser Kombination von Ablations- und Sensingelektrode ist es einerseits möglich, in einem einzigen Ablationsschritt aufgrund der linearen Ablationselektrode eine längliche Läsion zu erzeugen. Der wesentliche Nachteil, daß solche länglichen Elektroden normalerweise schlecht positionierbar sind, da sie entlang ihrer Längserstreckung keine ausreichende Wahrnehmungsfähigkeit bieten, wird durch die zusätzliche Sensingelektrode vermieden. Während bei einem Sensing mittels einer langen Elektrode die elektrische Erregungsfront bei Ausbreitung durch das Herzmuskelgewebe sich signaltechnisch entlang der langen Elektrode zu Null integriert, kann die in die Ablationselektrode eingebettete Sensingelektrode sehr kurz ausgeführt und damit hoch-wahrnehmungsfähig und ortsspezifisch sein.

Ein weiterer Vorteil der Verwendung einer einzigen, langgestreckten Ablationselektrode ist die Tatsache, daß ein herkömmlicher Standard-Ablationsgenerator mit einem Ausgang für die Bereitstellung der Ablationsenergie zur Erzeugung einer langgestreckten, linearen Läsion verwendet werden kann.

Bevorzugte Ausführungsformen des erfindungsgemäßen Ablationskatheters sind in den Unteransprüchen angegeben. Ferner sind weitere Merkmale, Einzelheiten und Vorteile der Erfindung der nachfolgenden Beschreibung entnehmbar, in der Ausführungsbeispiele des Erfindungsgegenstandes anhand der beigefügten Zeichnungen näher erläutert werden. Es zeigen:
- Fig. 1: eine Seitenansicht des distalen Endes eine Ablationskatheters in einer ersten Ausführungsform,
- Fig. 2: einen längsaxialen Schnitt durch den Ablationskatheter gemäß Fig. 1,
- Fig. 3 und 4: zwei längsaxiale, um 90° zueinander verdrehte Schnitte durch das distale Ende eines Ablationskatheters in einer zweiten Ausführungsform,
- Fig. 5: einen längsaxialen Schnitt durch das distale Ende eines Ablationskatheters in einer dritten Ausführungsform,
- Fig. 6A - B: radiale Schnitte durch das distale Ende entlang der Schnittlinie VI-VI nach Fig. 5 in aufeinanderfolgenden Fertigungszwischenschritten,
- Fig. 7 und 8: längsaxiale Schnitte des distalen Endes von Ablationskathetern in einer vierten und fünften Ausführungsform,
- Fig. 9: einen längsaxialen Schnitt durch das distale Ende eines Ablationskatheters in einer sechsten Ausführungsform,
- Fig. 10: einen längsaxialen Schnitt durch das distale Ende des Ablationskatheters gemäß Fig. 9 in einem Fertigungszwischenschritt, und
- Fig. 11: eine teilweise weggebrochene, perspektivische Darstellung des distalen Endes eines Ablationskatheters in einer siebten Ausführungsform.

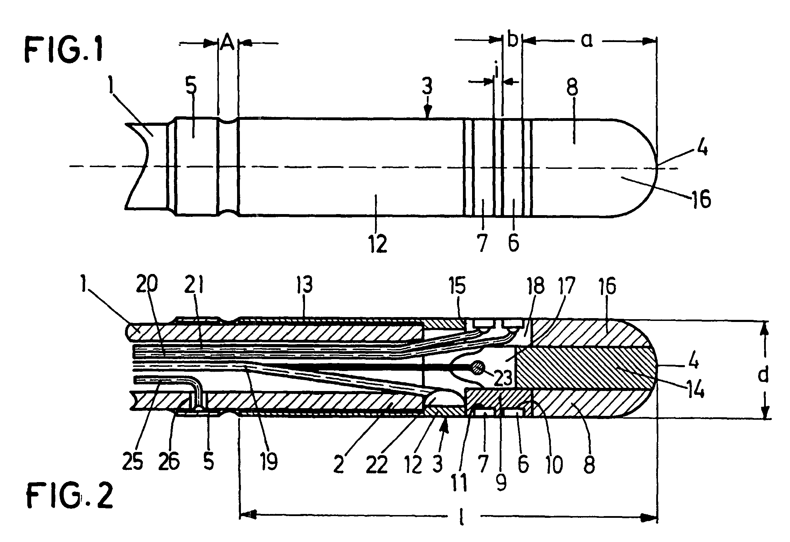

Anhand von Fig. 1 soll der grundsätzliche Aufbau eines Ablationskatheters erläutert werden. So ist ein langgestreckter, im Querschnitt kreisrunder Katheterkörper 1 vorgesehen, der in Fig. 1 und allen weiteren Zeichnungen lediglich rudimentär dargestellt ist. Er besteht aus einem physiologisch verträglichen, elastischen Kunststoffschlauch, dessen Durchmesser in der Größenordnung von 1 bis 2 mm liegt. An dem nicht gezeigten proximalen Ende des Katheterkörpers ist in üblicher Weise ein Handgriff mit entsprechenden Betätigungselementen zur Richtungssteuerung beim Einführen des Katheters in den Körper angeordnet.

Am distalen Ende 2 des Katheterkörpers 1 sitzt eine als Ganzes mit 3 bezeichnete Ablationselektrode, die eine langgestreckte, zylindrische Form mit abgerundeter Spitze aufweist. Charakteristische Größen für die Länge 1 der Ablationselektrode 3 und den Durchmesser d sind 1 = 10 mm und d=2,3 mm.

Etwa im mittleren Drittel der Ablationselektrode 3 sind zwei schmale, ringförmige Sensingelektroden 6, 7 in den Mantel 8 der Ablationselektrode 3 eingebettet angeordnet. Über einen Isolationsringkörper 9 sind dabei die beiden Sensingelektroden 6, 7 isoliert voneinander und auch von der Ablationselektrode 3 gelagert. Der Isolationsringkörper 9 weist zwei um den Umfang des Isolationsringkörpers 9 umlaufende Aufnahmenuten 10, 11 auf, deren Tiefe der Ringdicke der Sensingelektroden 6, 7 entspricht. Insoweit fluchten die außen sichtbaren Oberflächen der Ablationselektrode 3, der Sensingelektroden 6, 7 und des lsolationsringkörpers 9 miteinander.

Die distal gelegene Sensingelektrode 6 weist einen Abstand a von etwa 3 mm zur Spitze 4 auf. Zwischen den Sensingelektroden 6, 7 und zur Ablationselektrode 3 hin beträgt der Isolierabstand i, wie er durch den Isolationsringkörper 9 hergestellt wird, etwa 0,2 mm. Die Breite b der Sensingelektroden 6, 7 beträgt 0,5 mm.

In proximaler Richtung vor der Ablationselektrode 3 ist eine ringförmige Hilfs-Sensingelektrode 5 angeordnet, die mit einem axialen Abstand A von etwa 0,5 mm zur Ablationselektrode 3 isoliert auf dem Katheterkörper 1 sitzt. Die Sensingelektrode 5 dient dazu, bipolare Sensingsignale nicht nur zwischen den Sensingelektroden 6 und 7 abtasten zu können, sondern auch zwischen den Elektroden 6 und 5 oder 7 und 5, je nach Bedarf.

Wie aus Fig. 2 deutlich wird, ist in der dort gezeigten ersten Ausführungsform der Ablationselektrode 3 diese aus einem im wesentlichen hülsenförmigen Basisteil 12 gebildet, das der proximalen Richtung zugewandt ist und auf das distale Ende 2 des Katheterkörpers 1 aufgeschoben ist. Zur Fixierung dient eine geeignete Verklebung 13 zwischen Katheterkörper 1 und Basisteil 12. In distaler Richtung weist das Basisteil 12 eine zapfenförmige Verlängerung in Form eines Schaftes 14 auf, dessen Außendurchmesser im wesentlichen dem Innendurchmesser der Öffnung des Isolationsringkörpers 9 entspricht. Damit ist der Isolationsringkörper 9 auf den Schaft 14 vom distalen Ende her aufschiebbar, bis er an die umlaufende Ringschulter 15 des Basisteils 12 der Ablationselektrode 3 anschlägt. Auf den Schaft 14 wird dann ein Endkappenteil 16 der Ablationselektrode 3 aufgeschoben, die aus dem gleichen Material wie das Basisteil 12 - nämlich einer medizinischen Platin-Iridium-Legierung - besteht. In der in Fig. 2 gezeigten Montagestellung werden Endkappenteil 16 und Schaft 14 des Basisteils 12 durch Laserverschweißung an der Spitze 4 fest miteinander verbunden.

Wie aus Fig. 2 ferner hervorgeht, sind der Schaft 14 in seinem Ansatzbereich und die Ringschulter 15 sowie der Isolationsringkörper 9 jeweils mit einer flachen, schmalen, schlitzförmigen Aussparung 17, 18 versehen, die in Fig. 2 mit der Zeichnungsebene zusammenfällt. Durch diese Aussparungen 17 bzw. 17 und 18 hindurch sind die isolierten Anschlußleitungen 19, 20, 21 für die Ablationselektrode 3 bzw. die beiden Sensingelektroden 6, 7 geführt. Die Befestigung der nicht näher dargestellten Seelen der Anschlußleitungen 19, 20, 21 an den entsprechenden Elektroden 3, 6, 7 erfolgt über jeweilige Lötpunkte 22. Schließlich wird über eine Anschlußleitung 25 die Hilfs-Sensingelektrode 5 kontaktiert. Diese Anschlußleitung 25 ist über eine Öffnung 26 im Katheterkörper 1 nach außen geführt.

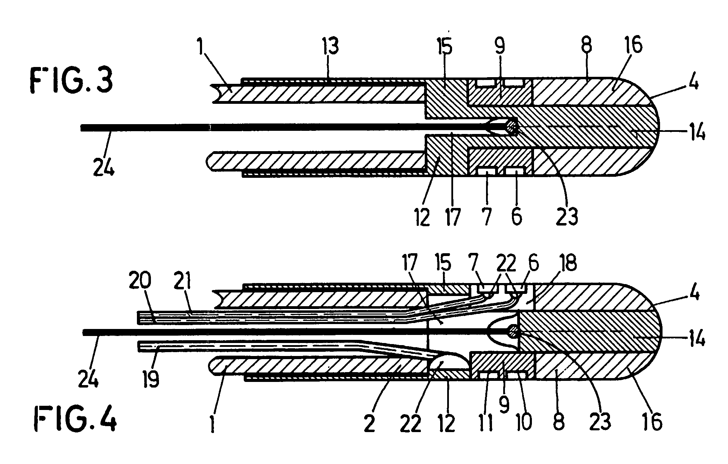

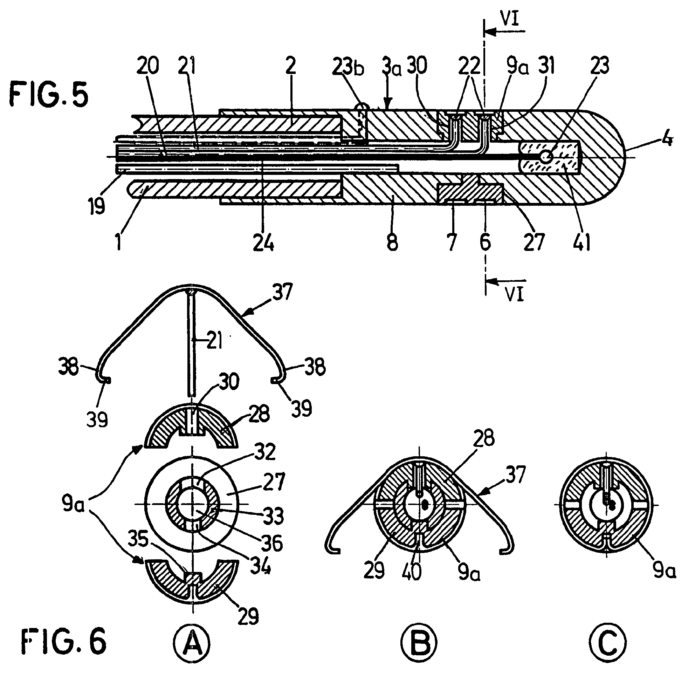

Die in Fig. 3 und 4 gezeigte Ausführungsform eines Ablationskatheters entspricht hinsichtlich der Ausführungsform der Ablationselektrode 3 mit Sensingelektroden 6, 7 der Variante gemäß den Fig. 1 und 2. Als Unterschied ist lediglich die Hilfs-Sensingelektrode 5 weggelassen. Insoweit erübrigt sich auch eine nochmalige Erörterung der in Fig. 3 und 4 dargestellten Ablationselektrode 3, übereinstimmende Bauelemente sind mit identischen Bezugszeichen versehen. Hinzuweisen ist allerdings auf Fig. 3, in der die Lage und Form der Aussparung 17 im Basisteil 12 durch einen Vergleich mit Fig. 4 bzw. Fig. 2 besonders deutlich wird.

Schließlich ist zu den Ausführungsformen gemäß Fig. 1 bis 4 festzuhalten, daß die Montage der Sensingelektroden 6, 7 in den Aufnahmenuten 10, 11 des einstückigen Isolationsringkörpers 9 analog der im folgenden beschriebenen Montageweise bei der Ausführungsform gemäß Fig. 5 und 6 erfolgen kann.

Bei der erwähnten Ausführungsform gemäß Fig. 5 und 6 ist auf den Katheterkörper 1 eine einstückige Ablationselektrode 3a mit zu Fig. 1 bis 4 übereinstimmender Außenkontur aufgesetzt. Etwa mittig weist nun die Ablationselektrode 3a eine tiefe Ringnut 27 auf, die wiederum zur Aufnahme des Isolationsringkörpers 9a dient. Letzterer ist in der Längsmittelebene zweigeteilt mit den Teilen 28, 29 ausgebildet und kann so von einander entgegengesetzten Seiten her in die Ringnut 27 eingesetzt werden, wie sich dies aus der Zusammenschau der Fig. 6A und 6B ergibt. Das in Fig. 5 und 6A oben liegende Teil 28 des Isolationsringkörpers 9a ist mit zwei radial verlaufenden Durchführungsöffnungen 30, 31 versehen, die radial nach innen verlängert und durch eine entsprechende radiale Durchgangsöffnung 32 im Kernring 33 am Boden der Ringnut 27 geführt sind. Auf der gegenüberliegenden Seite ist eine Zentrieröffnung 34 im Kernring 33 vorgesehen, die mit einer vom zweiten Isolierringkörper-Teil 29 nach innen vorspringenden Nase 35 zur Drehsicherung zusammenwirkt. Zentral in der Ablationselektrode 3a ist im übrigen eine Längsbohrung 36 vorgesehen, in die die Durchführungsöffnungen 30, 31 einmünden.

Wie nun aus Fig. 6 hervorgeht, werden die beiden Sensingelektroden 6, 7 jeweils aus einem entsprechend vorgeformten, in Seitenansicht V-förmigen Biegeteil 37 hergestellt, an das die jeweilige Anschlußleitung 20 bzw. 21 (Fig. 6A) nach der Durchfädelung durch den Katheterkörper 1 angelötet wird. Anschließend werden das Biegeteil 37 auf die in die Ringnut 27 eingesetzten Teile 28, 29 des Isolationsringkörpers 9a aufgesetzt (Fig. 6B) und anschließend die freien Enden 38 der beiden Schenkel des Biegeteils 37 um den Isolationsringkörper 9a im Umfangsrichtung herumgebogen, so daß das Biegeteil 37 in den entsprechenden Aufnahmenuten 10 bzw. 11 des Isolationsringkörpers 9a einliegt. An den freien Enden 38 des Biegeteils 37 sind ferner zwei kurze, einwärts gebogene Endstücke 39 vorgesehen, die beim Herumfalten des Biegeteils 37 in einen entsprechend geformten Schlitz 40 im unteren Teil 29 des Isolationsringkörpers 9a eintauchen (Fig. 6C). In dieser Stellung können die beiden Endstücke 39 miteinander laserverschweißt werden, wodurch die Sensingelektroden 6 bzw. 7 auf dem Isolationsringkörper 9a gesichert und letzterer gleichzeitig auf der Ablationselektrode 3 a fixiert ist.

Wie im übrigen aus Fig. 5 hervorgeht, ist über einen Klebstofftropfen 41 wiederum ein Thermoelement 23 mit Anschlußleitung 24 am distalen Ende der Längsbohrung 36 fixiert. Ferner ist strichliert in Fig. 5 ein an der Ablationselektrode 3a außen liegendes Thermoelement 23b dargestellt, das alternativ oder zusätzlich zum Thermoelement 23 einsetzbar ist. Dieses weitere Thermoelement 23b kann so beschaltet und an der Ablationselektrode montiert sein, daß es als zusätzliche elektrische Sensingelektrode dient.

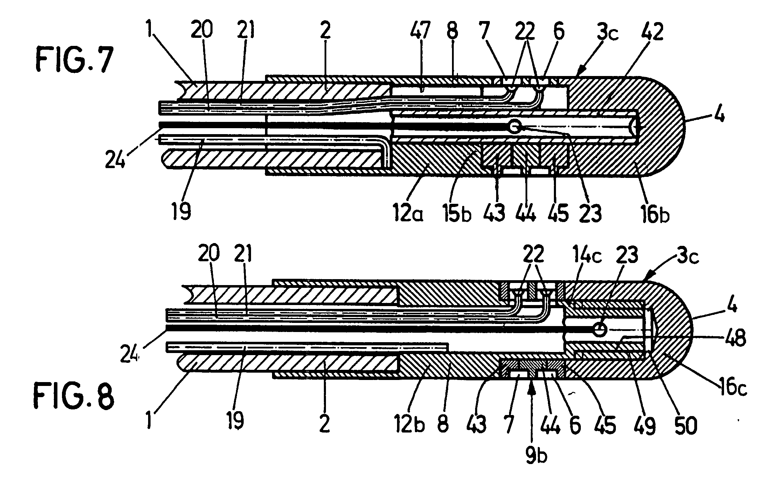

Bei der in Fig. 7 gezeigten Variante ist wiederum eine mehrteilige Ablationselektrode 3b vorgesehen, bei der das Basisteil 12a keinen direkt angeformten Schaft, sondern ein engeres Rohrstück 42 aus einer PtIr-Legierung aufweist. Auf dieses Rohrstück 42 ist der Isolationsringkörper 9b in Form mehrerer einzelner Teilringe 43, 44, 45 aufgesetzt, die durch peripher umlaufende Ringschultern 46 die Aufnahmenuten 10, 11 für die beiden Sensingelektroden 6, 7 bilden. Die Teilungsebene zwischen den Teilringen 43, 44, 45 liegen jeweils auf der Mittenebene der Aufnahmenuten 10, 11.

Zur Montage der Ablationselektrode 3b werden also nacheinander der Teilring 43, die Sensingelektrode 6, der Teilring 44, die Sensingelektrode 7 und schließlich der Teilring 45 auf das Rohrstück 42 aufgefädelt. Danach wird das Endkappenteil 16b ebenfalls auf das Rohrstück 42 aufgesteckt und verpreßt. Wahlweise können die einzelnen Teile auch mit Untermaß auf Preßsitz montiert werden und müssen dann nicht mehr abschließend verpreßt werden.

Die Kontaktierung der einzelnen Elektroden 3b, 6, 7 erfolgt wiederum über entsprechende Anschlußleitungen 19, 20, 21. Die Anschlußleitungen 20, 21 für die Sensingelektroden 6, 7 sind dabei in einer Längsnut 47 radial außerhalb des Rohrstückes 42 daran vorbei und zu den Sensingelektroden 6, 7 hingeführt. Die Anschlußleitung 19 für die Ablationselektrode 3b ist zwischen dem distalen Ende 2 des Katheterkörpers 1 und der Ringschulter 15b des Basisteils 12a nach außen geführt. Ferner ist wiederum ein Thermoelement 23 vorgesehen, das am Rohrstück 42 etwa mittig bezogen auf dessen Länge fixiert ist.

Die in Fig. 8 gezeigte Variante des Ablationskatheters weist eine Ablationselektrode 3b auf, die wiederum in ein Basisteil 12b und ein Endkappenteil 16c getrennt ist. An das Basisteil 12b ist ein Schaft 14c angeformt, der auf seiner Außenseite ein Gewinde 48 trägt. Dieses wirkt mit einem Innengewinde 49 einer in das Endkappenteil 16c eingebrachten Innengewindebohrung 50 zusammen. Insoweit kann die Ablationselektrode 3c wiederum durch Auffädeln der Teilringe 43, 44, 45 und Sensingelektroden 6, 7 auf den Schaft 14c und anschließendes Aufschrauben des Endkappenteils 16c montiert werden. Die weiteren Bauteile, wie Anschlußleitungen 19, 20, 21 etc., entsprechen den vorher beschriebenen Ausführungsformen.

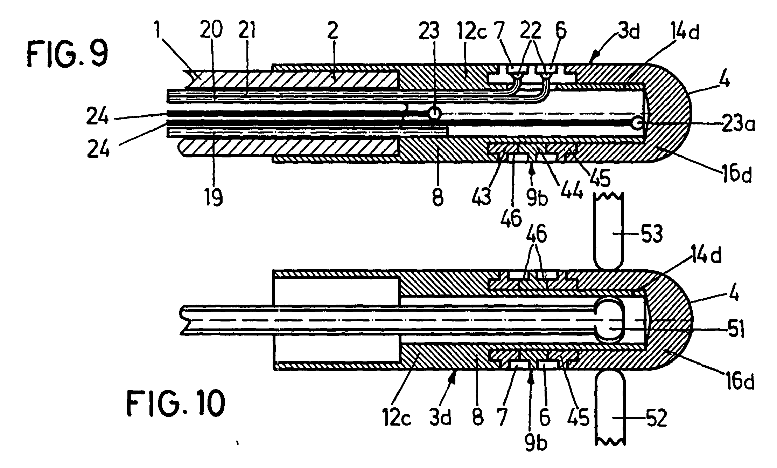

Die in Fig. 9 und 10 gezeigte Variante der Ablationselektrode 3d unterscheidet sich von der gemäß Fig. 8 lediglich dadurch, daß der Schaft 14d glatt ausgeführt ist und daß Endkappenteil 16d darauf aufgeschoben und widerstandsverschweißt ist. In Fig. 10 ist ein entsprechender Fertigungszwischenschritt dargestellt, bei dem Schweißelektroden 51, 52, 53 zwischen dem Endkappenteil 16d und der Durchgangsöffnung 32d im Schaft 14d angesetzt dargestellt sind.

Wie ferner aus Fig. 9 hervorgeht, sind bei dieser Variante zwei Thermoelemente 23, 23a mit entsprechenden Anschlußleitungen 24, 24a vorgesehen. Die Thermoelemente 23, 23a sind dabei jeweils im Bereich der Spitze 4 bzw. im Übergangsbereich zwischen Basisteil 12c und Katheterkörper 1 angeordnet.

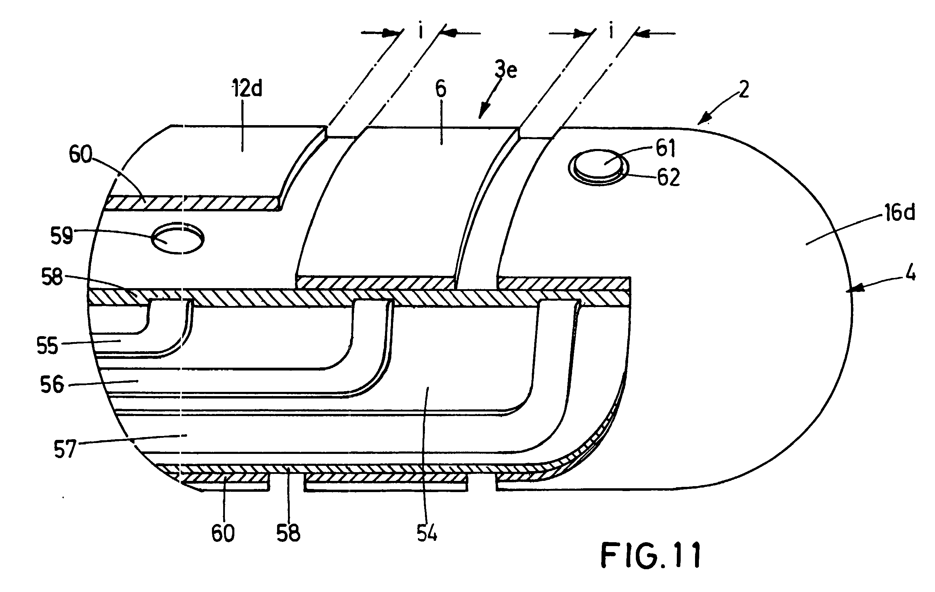

In Fig. 11 ist eine weitere Ausführungsform eines Ablationskatheters mit einer distalen Ablationselektrode 3e gezeigt, die auf der Basis einer auf dem Gebiet der Herstellung von elektronischen Komponenten bekannten 3-D-Strukturierungstechnologie hergestellt ist. Derartige Strukturierungstechnologien sind einerseits in Form von sogenannten laserselektiven Verfahren bekannt. Dabei wird eine Schicht, bei der es sich um einen Leiter oder auch einen Isolator handeln kann, in definierten Bereichen einer Laser-Abrasion unterworfen, wodurch entsprechende leitende oder isolierende Strukturen auf einem Substrat hergestellt werden. Andererseits sind 3-D-Strukturierungstechnologien auch auf der Basis einer Polymerisation elektrisch leitfähiger oder auch isolierender Pasten mittels lokal eingestrahlter Laserenergie einsetzbar. Letztere Technologie ist als Lasersintern bekannt.

Das distale Ende 2 der Ablationselektrode 3e weist nun einen den tragenden Kern der Ablationselektrode 3e bildenden, im wesentlichen zylindrischen Grundkörper 54 auf, der aus Keramik oder Kunststoff besteht bzw. dielekrische Eigenschaften aufweist. Dieser Grundkörper 54 ist für das distale Ende 2 geometriegebend und stellt die sogenannte Strukturebene 0 dar. Der guten Ordnung halber ist zu erwähnen, daß die "Ebene" im vorliegenden Ausführungsbeispiel natürlich zylindermantelförmig gewölbt ist.

Auf dem Grundkörper 54 sind elektrisch leitfähige Leiterbahnen 55, 56, 57 aufgebracht, die zur Kontaktierung der einzelnen, noch zu erläuternden Elektroden dienen. Diese Leiterbahnen sind in der ersten Strukturebene des Schichtaufbaus ausgebildet.

Unter Einschluß der Leiterbahnen 55, 56, 57 ist auf dem Grundkörper 54 anschließend eine Isolationsschicht 58 aufgebracht, die die zweite Strukturebene des Elektrodenaufbaus darstellt. Diese Isolationsschicht 58 besteht aus einem biokompatiblen Material und kann an geeigneten Stellen Durchbrüche 59 aufweisen, um eine Kontaktierungsmöglichkeit für die auf die Isolationsschicht 58 aufgebrachten elektrisch leitenden Schichten zur Ausbildung der einzelnen Elektrodenbestandteile zu schaffen. Diese als Ganzes mit 60 bezeichnete, strukturierte leitende Schicht ist so aufgebaut, daß daraus zum einen das Basisteil 12d der Ablationselektrode 3e gebildet wird, dessen elektrische Kontaktierung über den Durchbruch 59 durch die Leiterbahn 55 erfolgt. Mit einem Isolationsabstand i zu dem Basisteil 12d und dem Endkappenteil 16d, das ebenfalls aus einer entsprechenden leitenden Schicht ausgebildet ist, liegt eine ringförmige Sensingelektrode 6, die über nicht näher dargestellte Durchbrüche mit Hilfe der Leiterbahn 56 elektrisch angeschlossen ist.

In das Endkappenteil 16d der Ablationselektrode 3e ist eine punktförmige Sensingelektrode 61 mit einem isolierenden Ringspalt 62 zum Endkappenteil 16d eingepaßt. Weitere Punktelektroden können - wie in der Zeichnung nicht näher erkennbar ist - über den Umfang des Endkappenteils 16d verteilt angeordnet und zusammen mit der Punktelektrode 61 über die Leiterbahn 57 elektrisch angeschlossen sein. Die Kontaktleiterbahn des Endkappenteils 16d ist in Fig. 11 ebenfalls nicht erkennbar.

Die die dritte Strukturebene des Schichtaufbaus bildende elektrisch leitende Schicht 60, aus denen die verschiedenen Elektroden herausgebildet sind, ist wegen des stattfindenden Kontaktes zum Herzgewebe ebenfalls biokompatibel ausgelegt.

Neben dem bereits erwähnten Laserverfahren zur Oberflächenstrukturierung der Ablationselektrode 3e kann auch ein Kathodenstrahlbeschichtungsverfahren mit Verwendung einer entsprechend ausgebildeten Maske eingesetzt werden.

Abschließend ist ferner darauf hinzuweisen, daß das anhand des Ausführungsbeispiels gemäß Fig. 11 erläuterte 3-D-Strukturierungsverfahren zur Ausbildung der Elektrodenanordnung des Ablationskatheters auch bei anders konfigurierten und angelegten Elektroden einsetzbar und nicht an die in die Ablationselektrode eingebetteten Sensingelektroden gebunden ist.

Beispielsweise können Elektroden in orthogonaler Anordnung vorgesehen sein, um die Richtung der Reizleitung erfassen zu können, oder es liegen beabstandete Elektroden zur Messung der Reizleitungsgeschwindigkeit oder einfach der elektrischen Signale des Herzens an verschiedenen Orten des Myokard vor.

## Patentansprüche

1. Ablationskatheter zur Erzeugung linearer Läsionen in Herzmuskelgewebe mit
- einem langgestreckten Katheterkörper (1) mit einem proximalen und einem distalen Ende (2),
- einer linearen, im wesentlichen zylindrischen Ablationselektrode (3, 3a, 3b, 3c, 3d, 3e) am distalen Ende (2) des Katheterkörpers (1), und
- mindestens einer in der Ablationselektrode (3, 3a, 3b, 3c, 3d, 3e) davon isoliert angeordneten Sensingelektrode (6, 7, 61) zur Erfassung elektrokardiologischer Signale, **dadurch gekennzeichnet, daß** die mindestens eine Sensingelektrode (6, 7) als schmale Ringelektrode ausgebildet ist, die in den Mantel (8) des Ablationselektrode (3, 3a, 3b, 3c, 3d, 3e) mit Abstand (a) zur katheter-Spitze (4) isoliert eingebettet ist.

2. Ablationskatheter nach Anspruch 1, **dadurch gekennzeichnet, daß** mindestens zwei mit Abstand und isoliert voneinander an der Ablationselektrode (3, 3a, 3b, 3c, 3d, 3e) angeordnete Sensingelektroden (6, 7, 61) vorgesehen sind.

3. Ablationskatheter nach Anspruche 1 oder 2, **dadurch gekennzeichnet, daß** die mindestens eine Sensingelektrode (6, 7) in einem Isolationsringkörper (9, 9a, 9b) sitzt, der wiederum in einer im Mantel der Ablationselektrode (3, 3a, 3b, 3c, 3d) umlaufenden Ringnut (27) untergebracht ist.

4. Ablationskatheter nach Anspruch 3, **dadurch gekennzeichnet, daß** die Ablationselektrode (3a) einstückig ausgebildet ist, wobei
- der in der Ringnut (27) sitzende Isolationsringkörper (9a) zweiteilig ausgebildet und mit seinen beiden Teilen (28, 29) von einander entgegengesetzten Seiten in die Ringnut (27) eingesetzt ist und
- die mindestens eine Sensingelektrode (6, 7) als Biegeteil (37) in eine Aufnahmenut (10, 11) des Isolationsringkörpers (9a) eingesetzt und um den Umfang des Isolationsringkörpers (9a) herumgebogen ist.

5. Ablationskatheter nach Anspruch 4, **dadurch gekennzeichnet, daß** die Biegeenden (39) der mindestens einen Sensingelektrode (6, 7) miteinander verschweißt, vorzugsweise laserverschweißt sind.

6. Ablationskatheter nach einem der Ansprüche 1 bis 3, **dadurch gekennzeichnet, daß** die Ablationselektrode (3, 3b, 3c, 3d) aus einem der proximalen Richtung zugewandten Basisteil (12, 12b, 12c, 12d) und einem die distale Spitze (4) der Ablationselektrode (3, 3b, 3c, 3d) bildenden Endkappenteil (16, 16b, 16c, 16d) gebildet ist, wobei die mindestens eine Sensingelektrode (6, 7) und der aus mehreren Teilringen (43, 44, 45) zusammengesetzte Isolationsringkörper (9, 9b, 9c, 9d) auf einen zwischen Basis- und Endkappenteil (12, 12b, 12c, 12d; 16, 16b, 16c, 16d) angeordneten Schaft(14, 14b, 14c, 14d) aufgefädelt sind.

7. Ablationskatheter nach Anspruch 6, **dadurch gekennzeichnet, daß** der Schaft (14, 14b, 14c, 14d) einstückig mit dem Basisteil (12, 12b, 12c, 12d) der Ablationselektrode (3, 3b, 3c, 3d) ausgebildet ist.

8. Ablationskatheter nach einem der Ansprüche 1 bis 7, **dadurch gekennzeichnet, daß** die Ablationselektrode (3, 3a, 3b, 3c, 3d) in ihrem Inneren mit mindestens einem Thermoelement (23, 23a) zur Überwachung ihrer Temperatur versehen ist.

9. Ablationskatheter nach einem der Ansprüche 1 bis 8, **dadurch gekennzeichnet, daß** ein Thermoelement (23b) an der Außenseite der Abla tionselektrode (3a) angeordnet ist.

10. Ablationskatheter nach Anspruch 9, **dadurch gekennzeichnet, daß** das außenliegende Thermoelement (23a) gleichzeitig als Sensingelektrode fungiert.

11. Ablationskatheter mindestens nach Anspruch 2, **dadurch gekennzeichnet, daß** der Abstand (A) zwischen beiden Sensingelektroden (6, 7) zwischen 0,1 mm und 0,4 mm, vorzugsweise etwa 0,2 mm und die Breite (b) der Sensingelektroden (6, 7) zwischen 0,2 mm und 0,8 mm, vorzugsweise etwa 0,5 mm betragen.

12. Ablationskatheter mindestens nach Anspruch 1, **dadurch gekennzeichnet, daß** die Ablationselektrode (3e) und die Sensingelektroden (6, 61) schichtweise durch dreidimensionale Strukturierung auf einem Grundkörper (54) ausgebildet sind.

## Claims

1. An ablation catheter for producing linear lesions in the cardiac muscle tissue having
- an oblong catheter body (1) having proximal and distal ends (2),
- a linear, essentially cylindrical ablation electrode (3, 3a, 3b, 3c, 3d, 3e) at the distal end (2) of the catheter body (1), and
- at least one sensing electrode (6, 7, 61), positioned in the ablation electrode (3, 3a, 3b, 3c, 3d, 3e) and insulated therefrom, for detecting electrocardiological signals,
**characterized in that** the at least one sensing electrode (6, 7) is implemented as a narrow annular electrode, which is embedded insulated in the mantle (8) of the ablation electrode (3, 3a, 3b, 3c, 3d, 3e) at a distance (a) to the catheter tip (4).

2. The ablation catheter according to Claim 1, **characterized in that** at least two sensing electrodes (6, 7, 61) are provided on the ablation electrode (3, 3a, 3b, 3c, 3d, 3e) at a distance and insulated from one another.

3. The ablation catheter according to Claim 1 or 2, **characterized in that** the at least one sensing electrode (6, 7) is seated in an insulation annular body (9, 9a, 9 b), which is in turn housed in a peripheral annular groove (27) in the mantle of the ablation electrode (3, 3a, 3b, 3c, 3d, 3e).

4. The ablation catheter according to Claim 3, **characterized in that** the ablation electrode (3a) is implemented in one piece,
- the insulation annular body (9a) seated in the annular groove (27) being implemented in two parts and having its two parts (28, 29) inserted into the annular groove (27) from opposite sides and
- the at least one sensing electrode (6, 7) being inserted as a bent part (37) into a receiving groove (10, 11) of the insulation annular body (9a) and being bent around the circumference of the insulation annular body (9a).

5. The ablation catheter according to Claim 4, **characterized in that** the bent ends (39) of the at least one sensing electrode (6, 7) are welded to one another, preferably laser welded.

6. The ablation catheter according to one of Claims 1 through 3, **characterized in that** the ablation electrode (3, 3b, 3c, 3d) is formed by a base part (12, 12b, 12c, 12d) facing toward the proximal direction and an end cap part (16, 16b, 16c, 16d) forming the distal tip (4) of the ablation electrode (3, 3b, 3c, 3d), the at least one sensing electrode (6, 7) and the insulation annular body (9, 9b, 9c, 9d) assembled from multiple rings (43, 44, 45), being threaded onto a shaft (14, 14b, 14c, 14d) positioned between base part and end cap part (12, 12b, 12c, 12d; 16, 16b, 16c, 16d).

7. The ablation catheter according to Claim 6, **characterized in that** the shaft (14, 14b, 14c, 14d) is implemented in one piece with the base part (12, 12b, 12c, 12d) of the ablation electrode (3, 3b, 3c, 3d).

8. The ablation catheter according to one of Claims 1 through 7, **characterized in that** the ablation electrode (3, 3a, 3b, 3c, 3d) is provided in its interior with at least one thermocouple (23, 23a) for monitoring its temperature.

9. The ablation catheter according to one of Claims 1 through 8, **characterized in that** a thermocouple (23b) is positioned on the outside of the ablation electrode (3a).

10. The ablation catheter according to Claim 9, **characterized in that** the external thermocouple (23a) simultaneously functions as a sensing electrode.

11. The ablation catheter at least according to Claim 2, **characterized in that** the distance (A) between both sensing electrodes (6, 7) is between 0.1 mm and 0.4 mm, preferably approximately 0.2 mm, and the width (b) of the sensing electrodes (6, 7) is between 0.2 mm and 0.8 mm, preferably approximately 0.5 mm.

12. The ablation catheter at least according to Claim 1, **characterized in that** the ablation electrode (3e) and the sensing electrodes (6, 61) are implemented in layers through three-dimensional structuring on a main body (54).

## Revendications

1. Cathéter d'ablation pour la création de lésions linéaires dans le tissu du myocarde, comportant
- un corps de cathéter étiré en longueur (1) ayant une extrémité proximale et une extrémité distale (2),
- une électrode d'ablation (3, 3a, 3b, 3c, 3d, 3e) linéaire et sensiblement cylindrique à l'extrémité distale (2) du corps de cathéter (1) et
- au moins une électrode de détection (6, 7, 61) disposée de manière isolée par rapport à l'électrode d'ablation (3, 3a, 3b, 3c, 3d, 3e) pour enregistrer des signaux électrocardiologiques,
**caractérisé en ce que** l'au moins une électrode de détection (6, 7, 61) est réalisée sous forme d'une électrode annulaire étroite qui est noyée avec isolation dans la chemise (8) de l'électrode d'ablation (3, 3a, 3b, 3c, 3d, 3e) à distance (a) de la pointe du cathéter (4).

2. Cathéter d'ablation selon la revendication 1, **caractérisé en ce qu'**il est prévu au moins deux électrodes de détection (6, 7, 61) disposées à distance et isolées les unes des autres sur l'électrode d'ablation (3, 3a, 3b, 3c, 3d, 3e).

3. Cathéter d'ablation selon une des revendications 1 ou 2, **caractérisé en ce que** l'au moins une électrode de détection (6, 7, 61) est assise dans un corps annulaire d'isolation (9, 9a, 9b) qui est à son tour logé dans une rainure annulaire (27) périphérique dans la chemise de l'électrode d'ablation (3, 3a, 3b, 3c, 3d).

4. Cathéter d'ablation selon la revendication 3, **caractérisé en ce que** l'électrode d'ablation (3a) est réalisée d'une pièce, tandis que
- le corps annulaire d'isolation (9a) assis dans la rainure annulaire (27) est réalisé en deux pièces et est inséré par ses deux pièces (28, 29) par des côtés opposés dans la rainure annulaire (27 et que
- l'au moins une électrode de détection (6, 7) est insérée sous forme d'une pièce cintrée (37) dans une rainure réceptrice (10, 11) du corps annulaire d'isolation (9a) et est recourbée autour de la circonférence du corps annulaire d'isolation (9a).

5. Cathéter d'ablation selon la revendication 4, **caractérisé en ce que** les extrémités courbées (39) de l'au moins une électrode de détection (6, 7) sont soudées, de préférence soudées au laser, entre elles.

6. Cathéter d'ablation selon une des revendications 1 à 3, **caractérisé en ce que** l'électrode d'ablation (3, 3b, 3c, 3d) est constituée d'une pièce de base (12, 12b, 12c, 12d) tournée vers la direction proximale et d'une pièce de capuchon terminal (16, 16b, 16c, 16d) formant la pointe distale (4) de l'électrode d'ablation (3, 3b, 3c, 3d), tandis que l'au moins une électrode de détection (6, 7) et le corps annulaire d'isolation (9, 9b, 9c, 9d) composé de plusieurs segments d'anneau (43, 44, 45) sont enfilés sur un arbre (14, 14b, 14c, 14d) disposé entre la pièce de base et de capuchon terminal (12, 12b, 12c, 12d ; 16, 16b, 16c, 16d).

7. Cathéter d'ablation selon la revendication 6, **caractérisé en ce que** l'arbre (14, 14b, 14c, 14d) est réalisé d'une pièce avec la pièce de base (12, 12b, 12c, 12d) de l'électrode d'ablation (3, 3b, 3c, 3d).

8. Cathéter d'ablation selon une des revendications 1 à 7, **caractérisé en ce que** l'électrode d'ablation (3, 3a, 3b, 3c, 3d) est pourvue à l'intérieur d'au moins un thermoélément (23, 23a) pour surveiller sa température.

9. Cathéter d'ablation selon une des revendications 1 à 8, **caractérisé en ce qu'**un thermoélément (23b) est disposé sur la face extérieure de l'électrode d'ablation (3a).

10. Cathéter d'ablation selon la revendication 9, **caractérisé en ce que** le thermoélément (23a) placé à l'extérieur fait en même temps office d'électrode de détection.

11. Cathéter d'ablation au moins selon la revendication 2, **caractérisé en ce que** la distance (A) entre les deux électrodes de détection (6, 7) se situe entre 0,1 mm et 0,4 mm, de préférence à environ 0,2 mm, et que la largeur (b) des électrodes de détection (6, 7) se situe entre 0,2 et 0,8 mm, de préférence à environ 0,5mm.

12. Cathéter d'ablation au moins selon la revendication 1, **caractérisé en ce que** l'électrode d'ablation (3e) et les électrodes de détection (6, 61) sont réalisées par couches par structuration tridimensionnelle sur un corps de base (54).
